# EUROPEAN PATENT APPLICATION

(11) **EP 2 481 809 A1**
(43) Date of publication of application: **01.08.2012**
(21) Application number: 10818232.0
(22) Date of filing: 20.09.2010
(51) Int. Cl.: C12Q 1/68

(54) **REAGENT KIT FOR QUANTITATIVELY DETECTING THE MUTATIONS OF EPIDERMAL GROWTH FACTOR RECEPTOR (EGFR)**

(30) Priority: 22.09.2009 CN 200910176852
(71) Applicant: Beijing ACCB Biotech Ltd., Beijing 100094 (CN)
(72) Inventor: XU, Junpu, Beijing 100094 (CN); CHEN, Zhao, Beijing 100094 (CN); LI, Jun, Beijing 100094 (CN)
(74) Representative: Dossmann, Gérard
(86) International application number: PCT/CN2010/001445
(87) International publication number: WO 2011/035538

(57) **Abstract**

The present invention relates to a detection method and a detection kit for EGFR gene mutations, which relates to the therapeutic efficacy of molecular-targeted anti-cancer drugs. Particularly, the present invention relates to a fluorescent quantitative PCR method and kit for detecting mutations at hotspots of EGFR gene, together with the use thereof. The present invention detects the mutations at specific sites of EGFR gene, and can predict the therapeutic efficacy of EGFR tyrosine kinase inhibitors. Therefore, it can provide a guidance to individualize treatments for cancer patients.

SEQUENCE LISTING

<110> Beijing ACCB Biotech Ltd.

<120> KIT FOR QUANTITATIVELY DETECTING MUTATIONS OF EPIDERMAL GROWTH FACTOR RECEPTOR

<130> MUB12-2316EP

<160> 53

<170> PatentIn version 3.3

<210> 1
<211> 3633
<212> DNA
<213> Unknown

<220>
<223> Sequence of EGFR Exon (ref. NCBI NM_005228; positions 2155, 2235-2277 and 2573 are underlined)

<400> 1
atgcgaccct ccgggacggc cggggcagcg ctcctggcgc tgctggctgc gctctgcccg 60

gcgagtcggg ctctggagga aaagaaagtt tgccaaggca cgagtaacaa gctcacgcag 120

ttgggcactt ttgaagatca ttttctcagc ctccagagga tgttcaataa ctgtgaggtg 180

gtccttggga atttggaaat tacctatgtg cagaggaatt atgatctttc cttcttaaag 240

accatccagg aggtggctgg ttatgtcctc attgccctca acacagtgga gcgaattcct 300

ttggaaaacc tgcagatcat cagaggaaat atgtactacg aaaattccta tgccttagca 360

gtcttatcta actatgatgc aaataaaacc ggactgaagg agctgcccat gagaaattta 420

caggaaatcc tgcatggcgc cgtgcggttc agcaacaacc ctgccctgtg caacgtggag 480

agcatccagt ggcgggacat agtcagcagt gactttctca gcaacatgtc gatggacttc 540

cagaaccacc tgggcagctg ccaaaagtgt gatccaagct gtcccaatgg gagctgctgg 600

ggtgcaggag aggagaactg ccagaaactg accaaaatca tctgtgccca gcagtgctcc 660

gggcgctgcc gtggcaagtc ccccagtgac tgctgccaca accagtgtgc tgcaggctgc 720

acaggccccc gggagagcga ctgcctggtc tgccgcaaat tccgagacga agccacgtgc 780

aaggacacct gccccccact catgctctac aaccccacca cgtaccagat ggatgtgaac 840

cccgagggca aatacagctt tggtgccacc tgcgtgaaga agtgtccccg taattatgtg 900

gtgacagatc acggctcgtg cgtccgagcc tgtggggccg acagctatga gatggaggaa 960

gacggcgtcc gcaagtgtaa gaagtgcgaa gggccttgcc gcaaagtgtg taacggaata 1020

ggtattggtg aatttaaaga ctcactctcc ataaatgcta cgaatattaa acacttcaaa 1080

aactgcacct ccatcagtgg cgatctccac atcctgccgg tggcatttag gggtgactcc 1140

ttcacacata ctcctcctct ggatccacag gaactggata ttctgaaaac cgtaaaggaa 1200

atcacagggt ttttgctgat tcaggcttgg cctgaaaaca ggacggacct ccatgccttt 1260

gagaacctag aaatcatacg cggcaggacc aagcaacatg gtcagttttc tcttgcagtc 1320

gtcagcctga acataacatc cttgggatta cgctccctca aggagataag tgatggagat 1380

gtgataattt caggaaacaa aaatttgtgc tatgcaaata caataaactg gaaaaaactg 1440

tttgggacct ccggtcagaa aaccaaaatt ataagcaaca gaggtgaaaa cagctgcaag 1500

gccacaggcc aggtctgcca tgccttgtgc tcccccgagg gctgctgggg cccggagccc 1560

agggactgcg tctcttgccg gaatgtcagc cgaggcaggg aatgcgtgga caagtgcaag 1620

cttctggagg gtgagccaag ggagtttgtg gagaactctg agtgcataca gtgccaccca 1680

gagtgcctgc ctcaggccat gaacatcacc tgcacaggac ggggaccaga caactgtatc 1740

cagtgtgccc actacattga cggcccccac tgcgtcaaga cctgcccggc aggagtcatg 1800

ggagaaaaca acaccctggt ctggaagtac gcagacgccg gccatgtgtg ccacctgtgc 1860

catccaaact gcacctacgg atgcactggg ccaggtcttg aaggctgtcc aacgaatggg 1920

cctaagatcc cgtccatcgc cactgggatg gtgggggccc tcctcttgct gctggtggtg 1980

gccctgggga tcggcctctt catgcgaagg cgccacatcg ttcggaagcg cacgctgcgg 2040

aggctgctgc aggagaggga gcttgtggag cctcttacac ccagtggaga agctcccaac 2100

caagctctct tgaggatctt gaaggaaact gaattcaaaa agatcaaagt gctgggctcc 2160

ggtgcgttcg gcacggtgta taagggactc tggatcccag aaggtgagaa agttaaaatt 2220

cccgtcgcta tcaaggaatt aagagaagca acatctccga aagccaacaa ggaaatcctc 2280

gatgaagcct acgtgatggc cagcgtggac aacccccacg tgtgccgcct gctgggcatc 2340

tgcctcacct ccaccgtgca actcatcacg cagctcatgc ccttcggctg cctcctggac 2400

tatgtccggg aacacaaaga caatattggc tcccagtacc tgctcaactg gtgtgtgcag 2460

atcgcaaagg gcatgaacta cttggaggac cgtcgcttgg tgcaccgcga cctggcagcc 2520

aggaacgtac tggtgaaaac accgcagcat gtcaagatca cagattttgg gctggccaaa 2580

ctgctgggtg cggaagagaa agaataccat gcagaaggag gcaaagtgcc tatcaagtgg 2640

atggcattgg aatcaatttt acacagaatc tatacccacc agagtgatgt ctggagctac 2700

ggggtgaccg tttgggagtt gatgaccttt ggatccaagc catatgacgg aatccctgcc 2760

agcgagatct cctccatcct ggagaaagga gaacgcctcc ctcagccacc catatgtacc 2820

atcgatgtct acatgatcat ggtcaagtgc tggatgatag acgcagatag tcgcccaaag 2880

ttccgtgagt tgatcatcga attctccaaa atggcccgag acccccagcg ctaccttgtc 2940

attcaggggg atgaaagaat gcatttgcca agtcctacag actccaactt ctaccgtgcc 3000

ctgatggatg aagaagacat ggacgacgtg gtggatgccg acgagtacct catcccacag 3060

cagggcttct tcagcagccc ctccacgtca cggactcccc tcctgagctc tctgagtgca 3120

accagcaaca attccaccgt ggcttgcatt gatagaaatg ggctgcaaag ctgtcccatc 3180

aaggaagaca gcttcttgca gcgatacagc tcagacccca caggcgcctt gactgaggac 3240

agcatagacg acaccttcct cccagtgcct gaatacataa accagtccgt tcccaaaagg 3300

cccgctggct ctgtgcagaa tcctgtctat cacaatcagc ctctgaaccc cgcgcccagc 3360

agagacccac actaccagga cccccacagc actgcagtgg gcaaccccga gtatctcaac 3420

actgtccagc ccacctgtgt caacagcaca ttcgacagcc ctgcccactg ggcccagaaa 3480

ggcagccacc aaattagcct ggacaaccct gactaccagc aggacttctt tcccaaggaa 3540

gccaagccaa atggcatctt taagggctcc acagctgaaa atgcagaata cctaagggtc 3600

gcgccacaaa gcagtgaatt tattggagca tga 3633

<210> 2
<211> 200
<212> DNA
<213> Unknown

<220>
<223> a part of wild-type DNA sequence of EGFR 18(ref. NCBI NT_033968.5)

<400> 2
gctgaggtga cccttgtctc tgtgttcttg tcccccccag cttgtggagc ctcttacacc 60

cagtggagaa gctcccaacc aagctctctt gaggatcttg aaggaaactg aattcaaaaa 120

gatcaaagtg ctgggctccg gtgcgttcgg cacggtgtat aaggtaaggt ccctggcaca 180

ggcctctggg ctgggccgca 200

<210> 3
<211> 300
<212> DNA
<213> Unknown

<220>
<223> a part of wild-type DNA sequence of EGFR 19(ref. NCBI NT_033968.5)

<400> 3
cgtcacagcc cccagcaata tcagccttag gtgcggctcc acagccccag tgtccctcac 60

cttcggggtg catcgctggt aacatccacc cagatcactg ggcagcatgt ggcaccatct 120

cacaattgcc agttaacgtc ttccttctct ctctgtcata gggactctgg atcccagaag 180

gtgagaaagt taaaattccc gtcgctatca aggaattaag agaagcaaca tctccgaaag 240

ccaacaagga aatcctcgat gtgagtttct gctttgctgt gtgggggtcc atggctctga 300

<210> 4
<211> 300
<212> DNA
<213> Unknown

<220>
<223> a part of wild-type DNA sequence of EGFR 21(ref. NCBI NT_033968.5)

<400> 4
ttcttcccat gatgatctgt ccctcacagc agggtcttct ctgtttcagg gcatgaacta 60

cttggaggac cgtcgcttgg tgcaccgcga cctggcagcc aggaacgtac tggtgaaaac 120

accgcagcat gtcaagatca cagattttgg gctggccaaa ctgctgggtg cggaagagaa 180

agaataccat gcagaaggag gcaaagtaag gaggtggctt taggtcagcc agcattttcc 240

tgacaccagg gaccaggctg ccttcccact agctgtattg tttaacacat gcaggggagg 300

<210> 5
<211> 20
<212> DNA
<213> Artificial

<220>
<223> upstream primer of EGFR Exon 18

<400> 5
gaggatcttg aaggaaactg 20

<210> 6
<211> 19
<212> DNA
<213> Artificial

<220>
<223> upstream primer of EGFR Exon 18

<400> 6
ccagcttgtg gagcctctt 19

<210> 7
<211> 19
<212> DNA
<213> Artificial

<220>
<223> downstream primer of EGFR Exon 18

<400> 7
gccagggacc ttaccttat 19

<210> 8
<211> 21
<212> DNA
<213> Artificial

<220>
<223> downstream primer of EGFR Exon 18

<400> 8
ctgtgccagg gaccttacct t 21

<210> 9
<211> 19
<212> DNA
<213> Artificial

<220>
<223> upstream primer of EGFR Exon 19

<400>  9
cccagaaggt gagaaagtt                                                    19


<210>  10
<211>  20
<212>  DNA
<213>  Artificial

<220>
<223>  upstream primer of EGFR Exon 19

<400>  10
gggactctgg atcccagaag                                                   20


<210>  11
<211>  18
<212>  DNA
<213>  Artificial

<220>
<223>  downstream primer of EGFR Exon 19

<400>  11
cctgaggttc agagccat                                                     18


<210>  12
<211>  19
<212>  DNA
<213>  Artificial

<220>
<223>  downstream primer of EGFR Exon 19

<400>  12
cccacacagc aaagcagaa                                                    19


<210>  13
<211>  20
<212>  DNA
<213>  Artificial

<220>
<223>  upstream primer of EGFR Exon 21

<400>  13
gcagccagga acgtactggt                                                   20


<210>  14
<211>  20
<212>  DNA
<213>  Artificial

<220>
<223>  upstream primer of EGFR Exon 21

<400>  14
ccctcacagc agggtcttct                                                   20


<210>  15

<211>  18
<212>  DNA
<213>  Artificial

<220>
<223>  downstream primer of EGFR Exon 21

<400>  15
gtgggaaggc agcctggt                                                    18


<210>  16
<211>  18
<212>  DNA
<213>  Artificial

<220>
<223>  downstream primer of EGFR Exon 21

<400>  16
gtgggaaggc agcctggt                                                    18


<210>  17
<211>  21
<212>  DNA
<213>  Artificial

<220>
<223>  upstream primer of the G¡úA mutation at EGFR Exon 18 position 2155

<400>  17
tgctgagctc cggtgcgttc g                                                21


<210>  18
<211>  21
<212>  DNA
<213>  Artificial

<220>
<223>  downstream primer of the G¡úA mutation at EGFR Exon 18 position 2155

<400>  18
ggagctcagc actttgatct t                                                21


<210>  19
<211>  21
<212>  DNA
<213>  Artificial

<220>
<223>  upstream primer of the deletion at EGFR Exon 19 position 2235-2249

<400>  19
atcaaaacat ctccgaaagc c                                                21


<210>  20
<211>  21
<212>  DNA
<213>  Artificial

<220>

<223>  downstream primer of the deletion at EGFR Exon 19 position 2235-2249

<400>  20
atgttttgat agcgacggga a                                                21


<210>  21
<211>  21
<212>  DNA
<213>  Artificial

<220>
<223>  upstream primer of the deletion at EGFR Exon 19 position 2236-2250

<400>  21
tcaagacatc tccgaaagcc a                                                21


<210>  22
<211>  21
<212>  DNA
<213>  Artificial

<220>
<223>  downstream primer of the deletion at EGFR Exon 19 position 2236-2250

<400>  22
gatgtcttga tagcgacggg a                                                21


<210>  23
<211>  21
<212>  DNA
<213>  Artificial

<220>
<223>  upstream primer of the deletion at EGFR Exon 19 position 2254-2277

<400>  23
caacactcga tgtgagtttc t                                                21


<210>  24
<211>  21
<212>  DNA
<213>  Artificial

<220>
<223>  downstream primer of the deletion at EGFR Exon 19 position 2254-2277

<400>  24
tcgagtgttg cttctcttaa t                                                21


<210>  25
<211>  21
<212>  DNA
<213>  Artificial

<220>
<223>  upstream primer of the T¡úG mutation at EGFR Exon 21 position 2573

<400>  25
tgggcgggcc aaactgctgg g                                                21

<210>  26
<211>  21
<212>  DNA
<213>  Artificial

<220>
<223>  downstream primer of the T¡úG mutation at EGFR Exon 21 position 2573

<400>  26
tggcccgccc aaaatctgtg a                                                21


<210>  27
<211>  18
<212>  DNA
<213>  Artificial

<220>
<223>  wild-type probe of EGFR Exon 18

<400>  27
ggctccggtg cgttcggc                                                    18


<210>  28
<211>  21
<212>  DNA
<213>  Artificial

<220>
<223>  wild-type probe of EGFR Exon 18

<400>  28
cggagcccag cactttgatc t                                                21


<210>  29
<211>  19
<212>  DNA
<213>  Artificial

<220>
<223>  probe of the G¡úA mutation at EGFR Exon 18 position 2155

<400>  29
tgctgagctc cggtgcgtt                                                   19


<210>  30
<211>  22
<212>  DNA
<213>  Artificial

<220>
<223>  probe of the G¡úA mutation at EGFR Exon 18 position 2155

<400>  30
cggagctcag cactttgatc tt                                               22


<210>  31
<211>  25
<212>  DNA
<213>  Artificial

<220>
<223>  wild-type probe of EGFR Exon 19

<400>  31
tcaaggaatt aagagaagca acatc                                          25


<210>  32
<211>  26
<212>  DNA
<213>  Artificial

<220>
<223>  wild-type probe of EGFR Exon 19

<400>  32
cggagatgtt gcttctctta attcct                                         26


<210>  33
<211>  21
<212>  DNA
<213>  Artificial

<220>
<223>  probe of the deletion at EGFR Exon 19 position 2235-2249

<400>  33
cccgtcgcta tcaaaacatc t                                              21


<210>  34
<211>  21
<212>  DNA
<213>  Artificial

<220>
<223>  probe of the deletion at EGFR Exon 19 position 2235-2249

<400>  34
agatgttttg atagcgacgg g                                              21


<210>  35
<211>  22
<212>  DNA
<213>  Artificial

<220>
<223>  probe of the deletion at EGFR Exon 19 position 2236-2250

<400>  35
cccgtcgcta tcaagacatc tc                                             22


<210>  36
<211>  22
<212>  DNA
<213>  Artificial

<220>
<223>  probe of the deletion at EGFR Exon 19 position 2236-2250

<400>  36

gagatgtctt gatagcgacg gg                                                22

<210>  37
<211>  23
<212>  DNA
<213>  Artificial

<220>
<223>  probe of the deletion at EGFR Exon 19 position 2254-2277

<400>  37
aattaagaga agcaacactc gat                                               23

<210>  38
<211>  23
<212>  DNA
<213>  Artificial

<220>
<223>  probe of the deletion at EGFR Exon 19 position 2254-2277

<400>  38
atcgagtgtt gcttctctta att                                               23

<210>  39
<211>  20
<212>  DNA
<213>  Artificial

<220>
<223>  wild-type probe of EGFR Exon 21

<400>  39
tggccagccc aaaatctgtg                                                   20

<210>  40
<211>  23
<212>  DNA
<213>  Artificial

<220>
<223>  wild-type probe of EGFR Exon 21

<400>  40
aagatcacag attttgggct ggc                                               23

<210>  41
<211>  19
<212>  DNA
<213>  Artificial

<220>
<223>  probe of the T¡úG mutation at EGFR Exon 21 position 2573

<400>  41
tggcccgccc aaaatctgt                                                    19

<210>  42
<211>  21

<212>  DNA
<213>  Artificial

<220>
<223>  probe of the T¡úG mutation at EGFR Exon 21 position 2573

<400>  42
gatcacagat tttgggcggg c                                                21


<210>  43
<211>  18
<212>  DNA
<213>  Artificial

<220>
<223>  downstream primer of EGFR Exon 18

<400>  43
tgggatccag agtccctt                                                    18


<210>  44
<211>  20
<212>  DNA
<213>  Artificial

<220>
<223>  downstream primer of EGFR Exon 19

<400>  44
gtctttgtgt tcccggacat                                                  20


<210>  45
<211>  20
<212>  DNA
<213>  Artificial

<220>
<223>  downstream primer of EGFR Exon 21

<400>  45
gcctccttct gcatggtatt                                                  20


<210>  46
<211>  18
<212>  DNA
<213>  Unknown

<220>
<223>  nucleic acid sequence containing G at EGFR Exon 18 position 2155
(position 2155 is underlined)

<400>  46
aaagtgctgg gctccggt                                                    18


<210>  47
<211>  18
<212>  DNA
<213>  Unknown

<220>

<223>  nucleic acid sequence containing A at EGFR Exon 18 position 2155
(position 2155 is underlined)

<400>  47
aaagtgctgg gctccggt                                                    18


<210>  48
<211>  53
<212>  DNA
<213>  Unknown

<220>
<223>  nucleic acid sequence containing wild-type sequence at EGFR Exon 18
position 2235-2277 (position 2235-2277 is underlined)

<400>  48
tcaaggaatt aagagaagca acatctccga aagccaacaa ggaaatcctc gat             53


<210>  49
<211>  38
<212>  DNA
<213>  Unknown

<220>
<223>  nucleic acid sequence containing the deletion at EGFR Exon 19
position 2235-2249

<400>  49
cgctatcaaa acatctccga aagccaacaa ggaaatcc                              38


<210>  50
<211>  39
<212>  DNA
<213>  Unknown

<220>
<223>  DNA sequence containing the deletion at EGFR Exon 19 position
2236-2250

<400>  50
cgtcgctatc aagacatctc cgaaagccaa caaggaaat                             39


<210>  51
<211>  35
<212>  DNA
<213>  Unknown

<220>
<223>  DNA sequence containing the deletion at EGFR Exon 19 position
2254-2277

<400>  51
aagagaagca acactcgatg tgagtttctg ctttg                                 35


<210>  52
<211>  18
<212>  DNA
<213>  Unknown

<220>

<223>  DNA sequence containing T at EGFR Exon 21 position 2573 (position
2573 is underlined)

<400>  52
acagattttg ggctggcc                                                    18


<210>  53
<211>  18
<212>  DNA
<213>  Unknown

<220>
<223>  DNA sequence containing G at EGFR Exon 21 position 2573 (position
2573 is underlined)

<400>  53
acagattttg ggcgggcc                                                    18

## Description

### BACKGROUND OF THE INVENTION

Epidermal Growth Factor Receptor (EGFR), a multifunctional glycoprotein widely spread on cell membranes of human tissues, is a member of HER/ErbB family, and is related with tumor propagation, vascular-genesis, tumor metastasis and resistance to apoptosis.

Most of the tumor cells express EGFR and its natural ligand, which, after binding to each other, can cause self phosphorylation, and transfer signal into nucleus via series of reactions, thus influence tumor development and evolution by influencing the growth and apoptosis of tumor cells, and tumor vascular-genesis.

It has been reported that EGFR plays an important role in cancer initiation and development. Targeting drugs directed to EGFR, e.g. EGFR tyrosine kinase inhibitors (EGFR-TKIs) such as AVASTIN, Erlotinib and Irressa etc., can inhibit propagation, invasion, metastasis of tumor cells and vascular-genesis, and induce apoptosis of tumor cells, by blocking EGFR signal transduction in tumor cells.

However, clinical practice shows that only 8-18% of Non-small-cell lung carcinoma patient can benefit from EGFR TKIs such as Iressa etc. (Paez JG, et al. Science , 2004 , 304: 1497-1500 ; Sequist LV, et al, Oncologist, 2007, 12(1):90-8). It is found that the therapy effect is significant for patients carrying EGFR gene mutation. The frequency of mutations is consistent to the population's sensitivity to Iressa: more women than men; more non-smokers than smokers; more adenocarcinomas than others, and more Eastern people than Western people. By analyzing the mutations of EGFR gene in Iressa sensitive patient tissues, it was found that most individuals had mutations in the EGFR gene tyrosine kinase region. These mutations mainly exist in Exon 18-21 (Chan SK, et al, Eur J Cancer, 2006, 42(1): 17-23). Therefore, by detecting the mutations in EGFR gene Exon 18-21, it is possible to predict the therapy effect of molecular-targeted drugs such as Iressa. Taking in consideration of the most common sites of EGFR mutations in Chinese population (Han Yu et al., Chinese Oncology Journal, 2007, 29(4):278-282; Guo Jian et al., Chinese Lung-Cancer Journal, 2007, 10(6): 504-507) and using real-time quantitative PCR, the present invention detects five kinds of mutations in EGFR Exon 18, 19 and 21, which are related to the therapy efficacy of molecular-targeted anti-tumor drugs EGFR-TKIs. The therapy efficacy of EGFR-TKIs will be predicted by using the method described in the present invention for detecting the EGFR gene mutations.

The detecting method of the present invention has the following advantages: easy manipulation, and easy standardization. Other methods, such as allele specific oligonucleotide probe hybridization method, are very much dependent on hybridization conditions, thus they need strict controlling of the experimental conditions. The restriction fragment length polymorphism method, on the other hand, needs a lot of human labor, and can not generate quantitative results. The method of the present invention has short experimental cycle, and can be completed with 2 hours. It doesn't need to verify the result by sequencing, whereas the direct sequencing and high resolution melting analysis need 4 days to 2 weeks. Sensitivity of the method of the present invention is high, which, after optimizing experimental conditions, can reach 1% for detecting mutations, whereas sensitivity of direct sequencing is 20-50%. Specificity of the method of the present invention is also high. Immunohistochemistry (IHC) method can easily get pseudo-positive and pseudo-negative results, and can not determine the position and types of point mutations. The unique advantage of the present invention is accurate quantification. By using absolute quantification method to analyze data, draw standard curve, and accurately determine the content of wild-type gene and mutant gene in the samples, one can obtain ratio of the mutant gene in the samples, which will be of help to clinical diagnosis and therapeutic selection. Further, the present invention is safe and non-toxic, other methods such as chemical breaking method of mismatch base need isotope and toxic chemical agents.

### SUMMARY OF THE INVENTION

The question that the present invention addresses is to provide a quantitative detection kit for EGFR gene mutations, which can quantitatively detect the following mutations: EGFR Exon 18 (SEQ ID NO:1; SEQ ID NO:2) position 2155 G substituted with A; Exon 19 (SEQ ID NO:1; SEQ ID NO:3) position 2235-2249 deletion; Exon 19 position 2236-2250 deletion; Exon 19 2254-2277 deletion; and Exon 21 (SEQ ID NO:1 ; SEQ ID NO:4) position 2573 T substituted with G.

To address the above question, the present invention providers quantitative detection kit containing a mixture comprising Taq enzyme, 10 x Taq buffer, MgCl₂, dNTP mixture, PCR primers which can specifically amplify the sequences at EGFR gene mutation positions, and probes which can specifically identify wild-type sequences and mutant sequences, together with method of the detection.
(1) Separately design upstream and downstream primers around the mutation positions of Exon 18, 19 and 21 of EGFR gene; and design specific probes according to each mutant site. Said probes can specifically bind wild-type sequences or the mutant sequences to be detected at specific EGFR sites, so as to determine wether the tested mutations occur at said sites.
(2) To accurately and quantitatively determine the ratio of the EGFR mutations, standards were designed in the present invention.
(3) Use fluorescent quantitative PCR to detect the samples and standards.
(4) Obtain standard curves for quantitative detection from the detection results of the standards, and calculate the ratios of EGFR gene mutations to the total wild type EGFR gene in the samples to be tested.

Prior to said step (1) it further includes: extracting nucleic acid from the samples, purifying it and determining the content of it.

The probes for fluorescent quantitative PCR specifically bind the sequences at EGFR gene mutation sites under suitable PCR conditions. Preferably, said probes link a fluorescence emitting group at their 5' end, and link a fluorescence quencher group at their 3' end. Said fluorescence emitting group is selected from FAM, TET, HEX and ROX. Said fluorescence quencher group is selected from BHQ, TAMARA. Preferably, said emitting group is FAM, and said quencher group is BHQ. Preferably, the sequences of said probes are SEQ ID NO: 27, SEQ ID NO: 28, SEQ IT NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41 or SEQ ID NO: 42.

Said standards include at least one of plasmids, genome DNA or chemically synthesized sequences. Preferably, said standards comprises a wild-type plasmid, a mutant plasmid, or both a wild-type plasmid and a mutant plasmid, wherein said wild-type plasmids include wild-type sequences of EGFR gene, and said mutant plasmids include mutant sequences of EGFR gene. More preferably, said standards are consisted of a wild-type plasmid, a mutant plasmid, or both a wild-type plasmid and a mutant plasmid. Preferably, the wild-type sequences of EGFR gene included in said wild-type plasmids are SEQ ID NO:46, SEQ ID NO:48 or SEQ ID NO:52, and the mutant sequences of EGFR gene included in said mutant plasmids are SEQ ID NO:47, SEQ ID NO:49, SEQ ID NO:50, SEQ ID NO:51 or SEQ ID NO:53.

The tested samples include fresh tissue, paraffin embedded tissues, cell lines, blood, pleural effusion, peritoneal effusion, saliva, digestive juice, urine and feces.

Said primers are consisted of upstream primers and downstream primers. Preferably, said primers are SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15 or SEQ ID NO: 16.

Said quantitative detection kit for EGFR gene mutations includes the agents selected from: the above-mentioned primers, probes and standards. Preferably, said kit further includes Taq enzyme, 10 x Taq buffer, MgCl₂, and dNTP mixture. Preferably, the ratio of primers to probes is 2:1-10:1, and the ratio of forward primers to reverse primers is 1:3-3:1. Said standards include a mixture of said plasmids in a certain ratio, wherein the ratio of the content of wild-type plasmids to mutant plasmids is 0%-100%.

### BRIEF DESCRIPTION OF DRAWINGS

The accompanying drawings, which are incorporated in and form a part of this specification, illustrate embodiments of the technology and, together with the description.

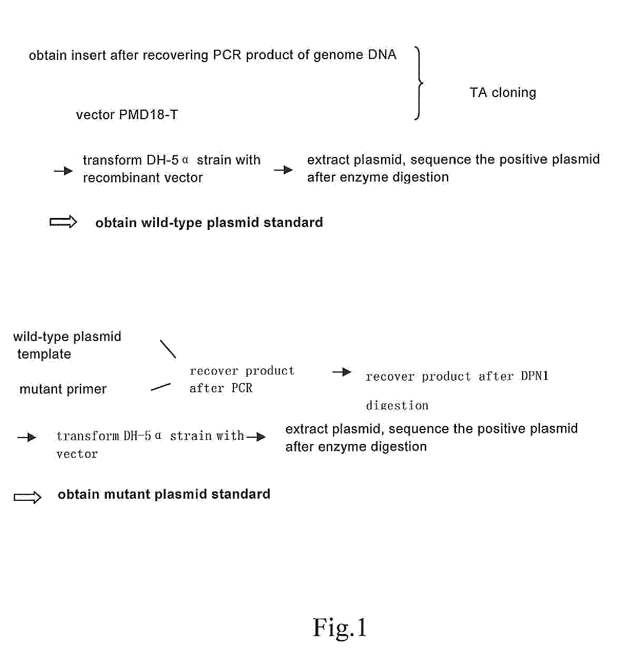

Figure 1 is a diagram showing the method for constructing the plasmid standards in Example 2.

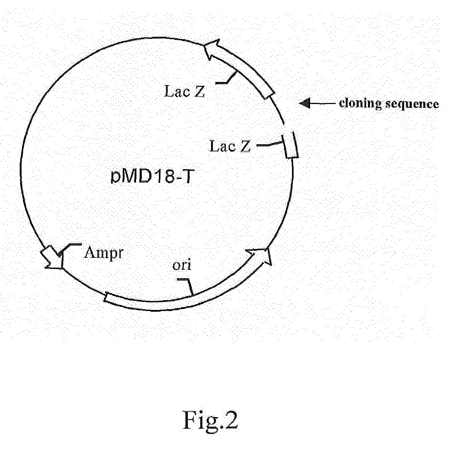

Figure 2 is a diagram showing the wild-type plasmid profile of Example 2, wherein the wild-type PCR product sequence is inserted into the carrier at the position marked with an arrow.

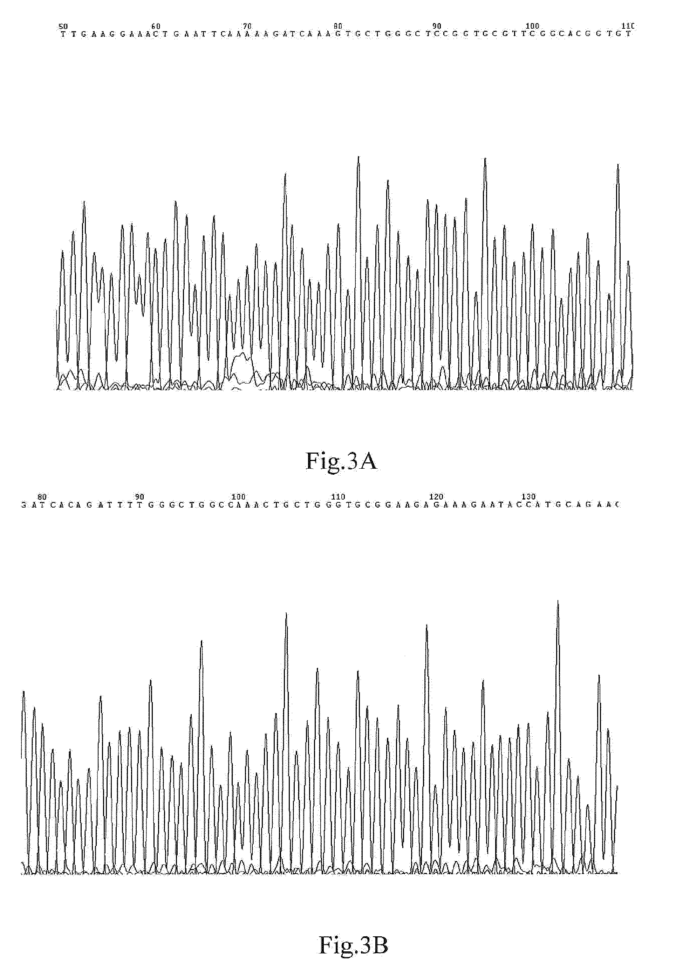

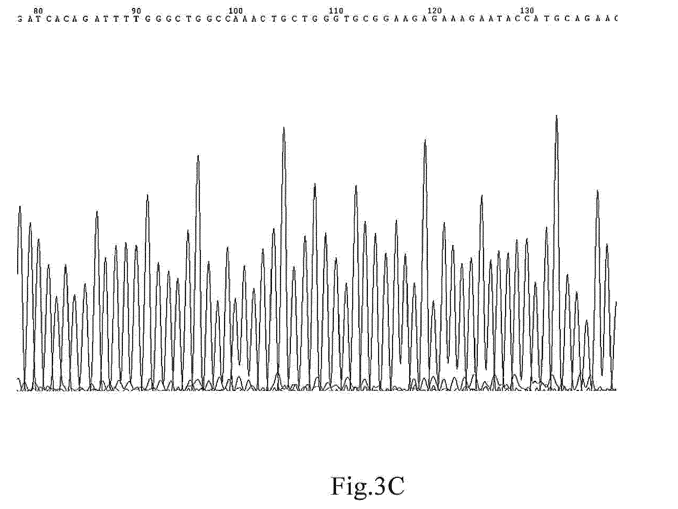

Figure 3 is a diagram showing the result of sequencing the wild-type plasmid standard of Example 2, wherein Fig.A is the sequencing result of EGFR Exon 18 2155G (wild-type) plasmid, Fig.B is the sequencing result of plasmid in which position 2235-2249, 2236-2250 and 2254-2277 of EGFR Exon 19 being wild-type, Fig.C is the sequencing result of plasmid in which position 2573 of EGFR Exon 21 being T (wild-type).

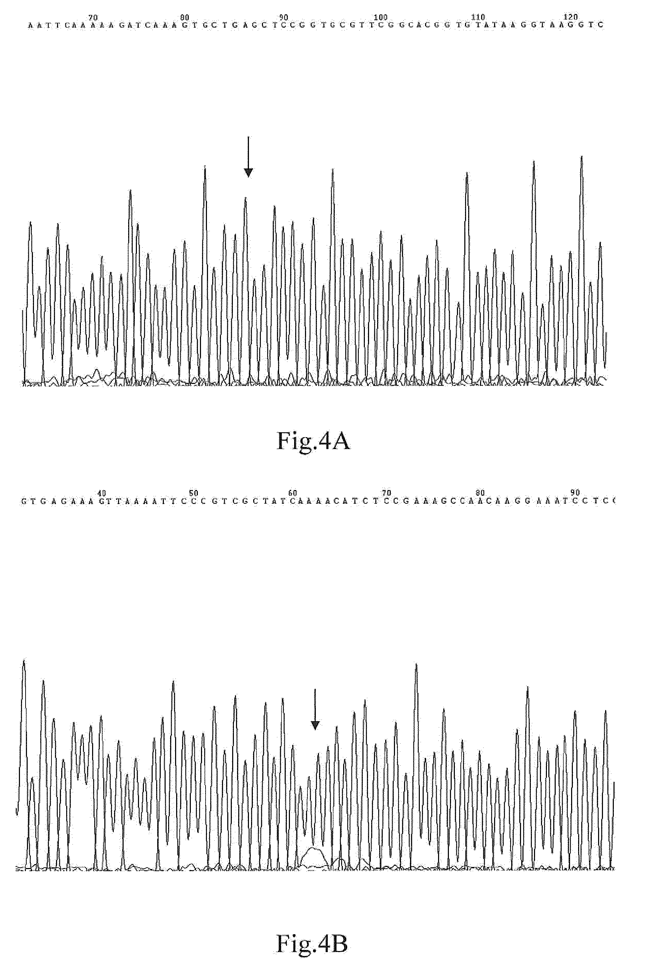

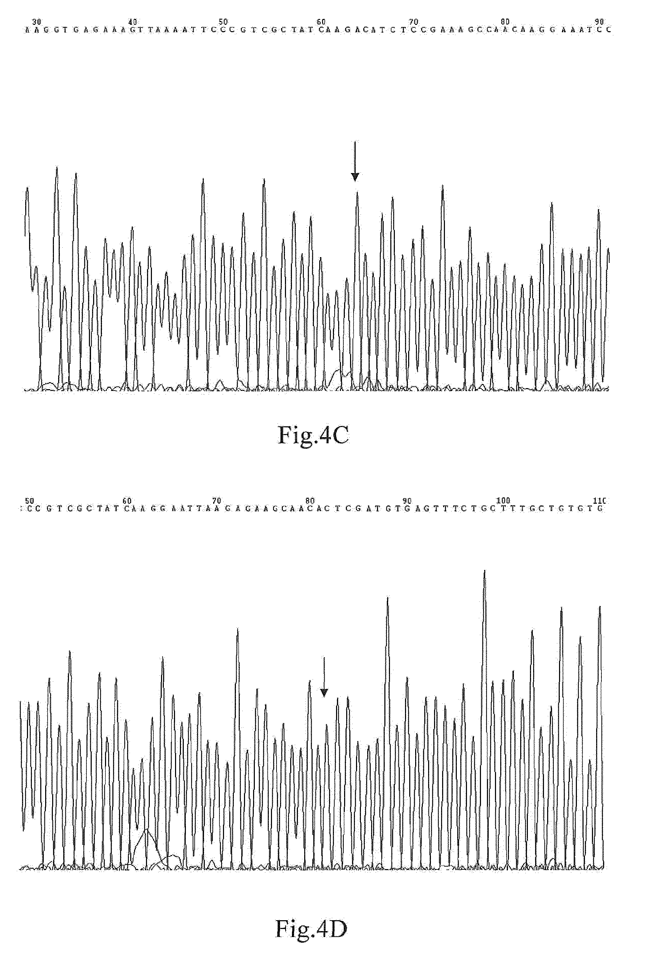

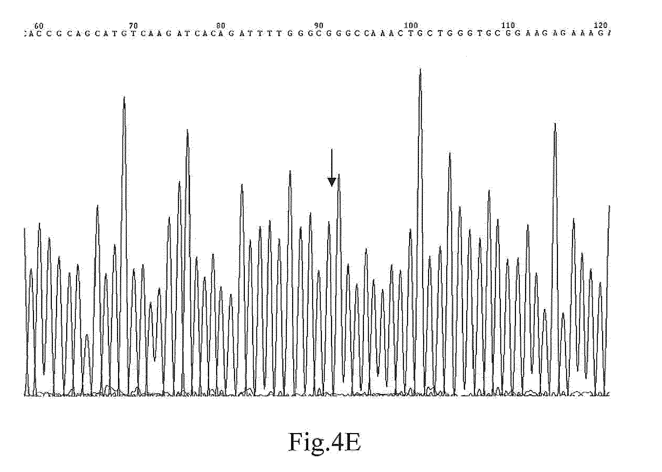

Figure 4 is a diagram showing the sequencing result of mutant plasmid standard of Example 2, wherein the mutant site is marked with an arrow. Fig.A, shows the sequencing of EGFR Exon 18 mutant of position 2155 G→A; Fig.B shows the sequencing of EGFR Exon 19 mutant of position 2235-2249 deletion; Fig.C shows the sequencing of EGFR Exon 19 mutant of position 2236-2250 deletion; Fig.D shows the sequencing of EGFR Exon 19 mutant of position 2254-2277 deletion; and Fig.E shows the sequencing of EGFR Exon 21 mutant of position 2573 T→G.

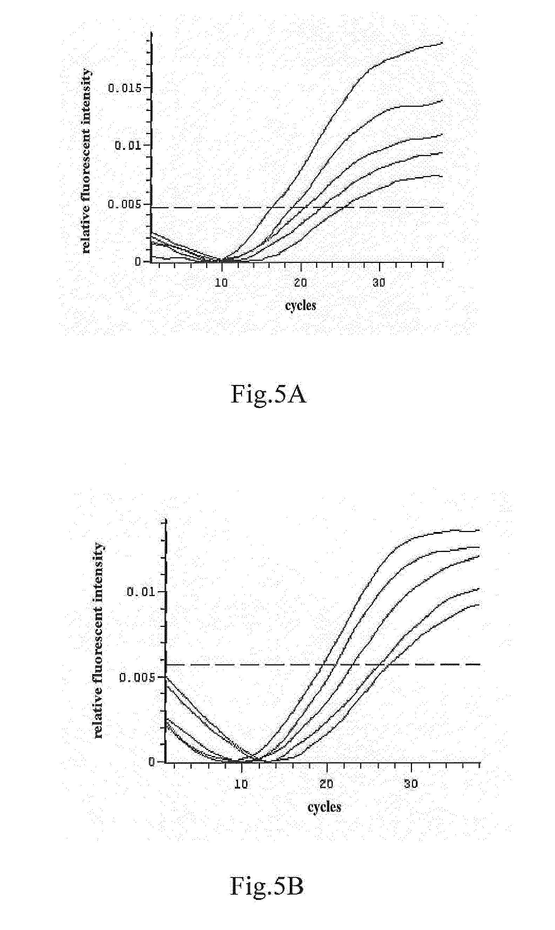

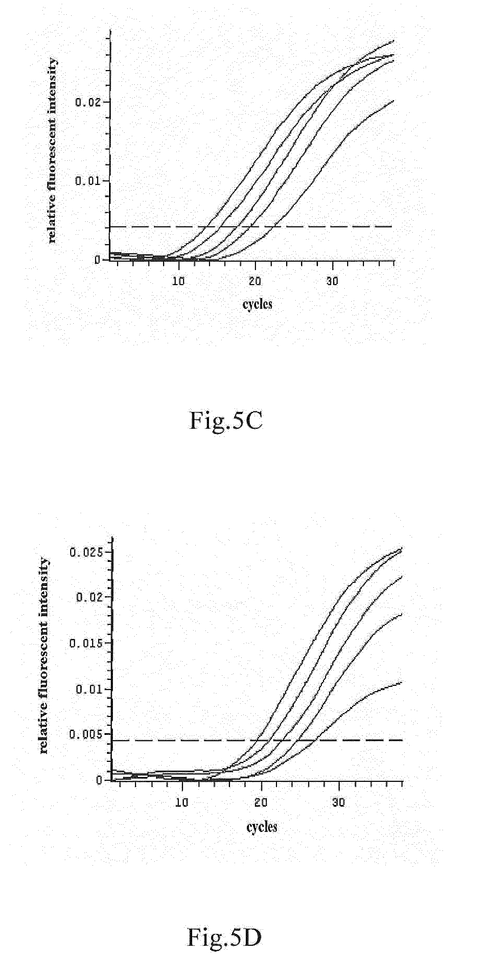

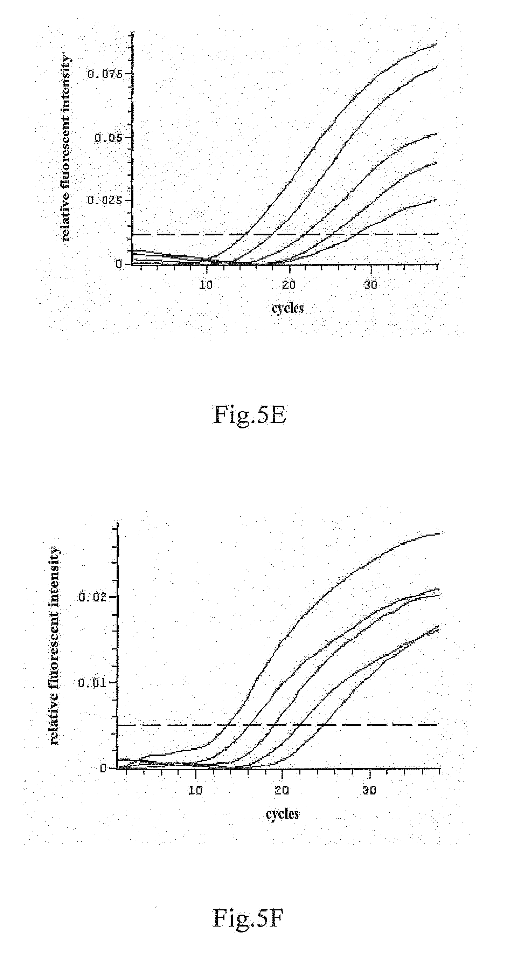

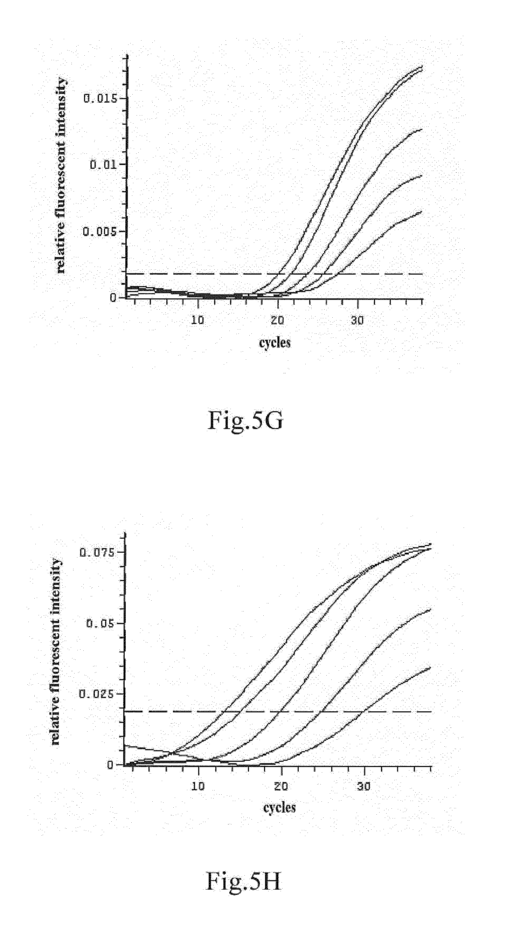

Figure 5 shows the amplification curve of the standard of Example 3, wherein Fig.A shows the amplification curve of the plasmid standard of EGFR Exon 18 2155G (wild-type); Fig.B shows the amplification curve of the plasmid standard of EGFR Exon 19 position 2235-2249, 2236-2250 and 2254-2277 being wild-type; Fig.C shows the amplification curve of the plasmid standard of EGFR Exon 21 position 2573 being T(wild-type); Fig.D shows the amplification curve of mutant plasmid standard of EGFR Exon 18 position 2155 being G→A; Fig.E shows the amplification curve of the mutant plasmid standard of EGFR Exon 19 position 2235-2249 deletion; Fig.F shows the amplification curve of mutant plasmid standard of EGFR Exon 19 position 2236-2250 deletion; Fig.G shows the amplification curve of mutant plasmid standard of EGFR Exon 19 position 2254-2277 deletion; Fig.H shows the amplification curve of mutant plasmid standard of EGFR Exon 19 position 2573 being T→G.

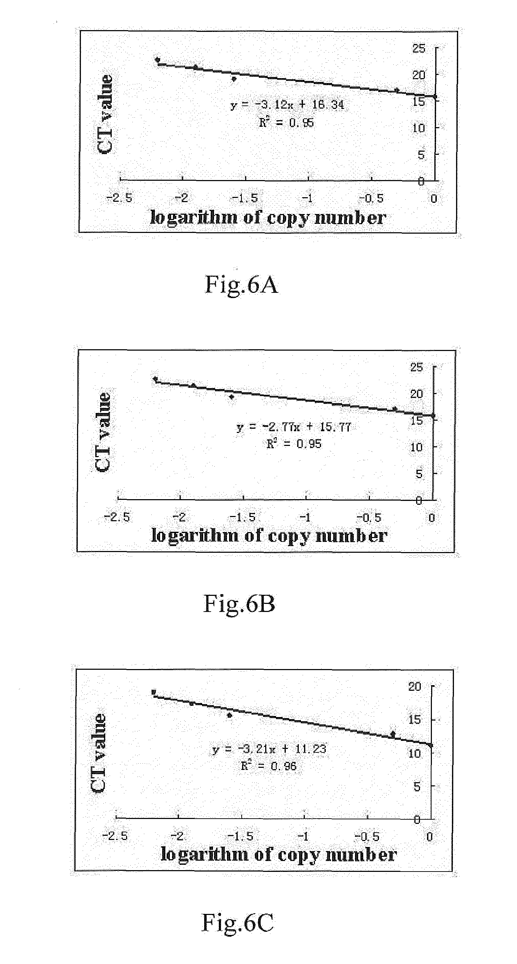

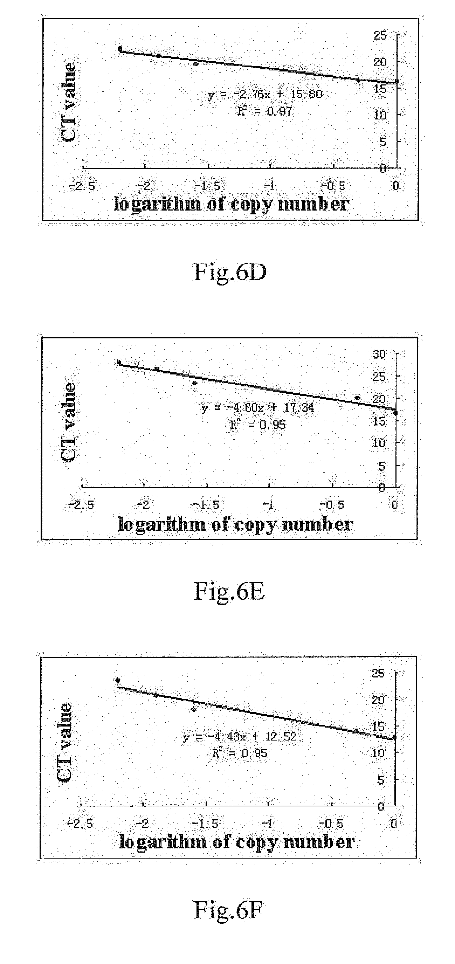

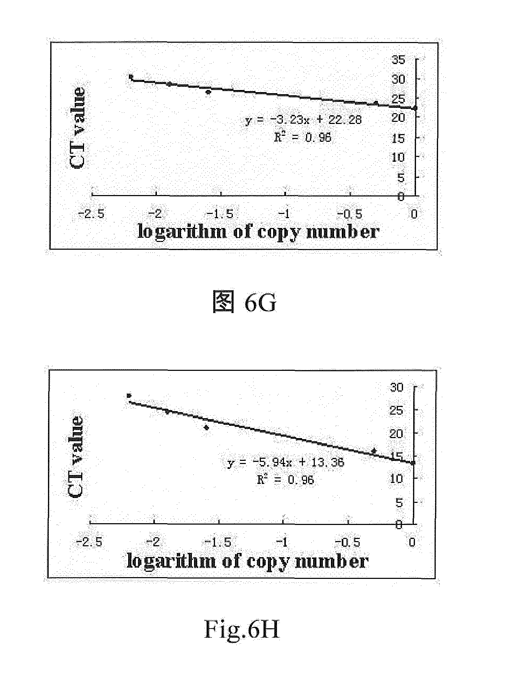

Figure 6 shows a standard curve based on Figure 4, wherein Fig.A is a standard curve of plasmid with EGFR Exon 18 2155G (wild-type); Fig.B is a standard curve of plasmid with EGFR Exon 19 position 2235-2249, 2236-2250 and 2254-2277 being wild-type; Fig.C is a standard curve of plasmid with EGFR Exon 19 position 2473 being T (wild-type); Fig.D is a standard curve of mutant plasmid with EGFR Exon 18 position 2155 being G→A; Fig.E is a standard curve of mutant plasmid with EGFR Exon 19 position 2235-2249 being deletion; Fig.F is a standard curve of plasmid with EGFR Exon 19 position 2236-2250 being deletion; Fig.G is a standard curve of plasmid with EGFR Exon 19 position 2254-2277 being deletion; Fig.H is a standard curve of plasmid with EGFR Oxon 18 position 2573 being T→G.

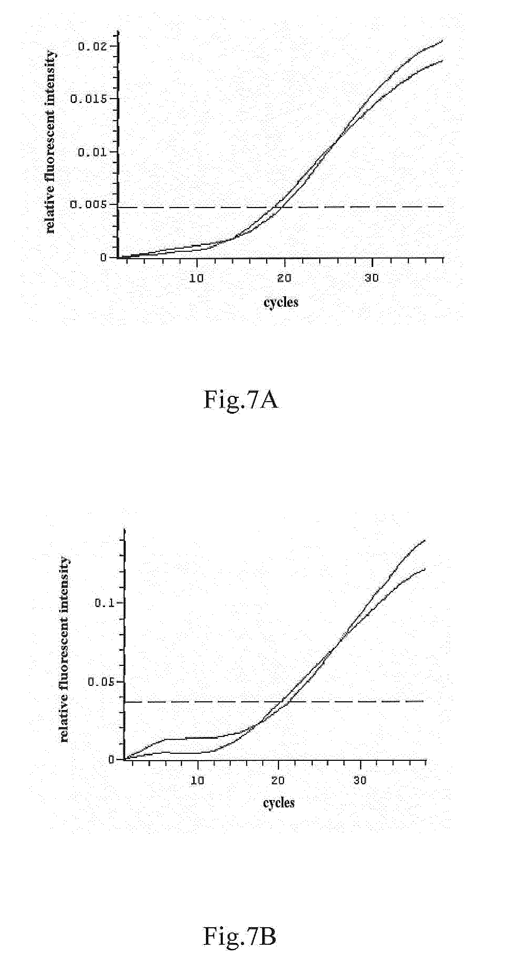

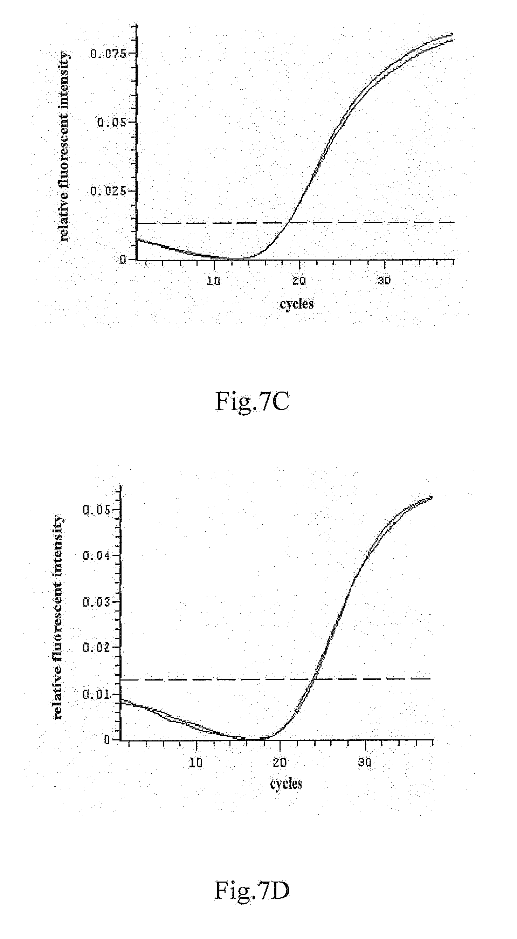

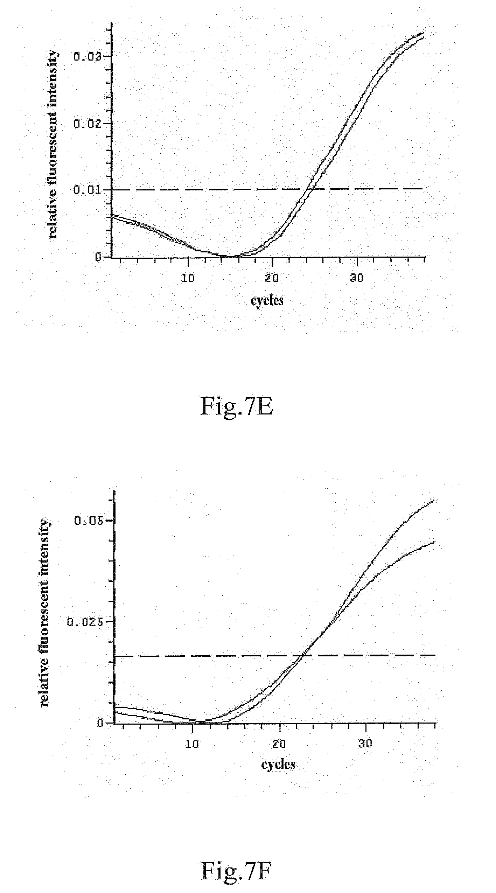

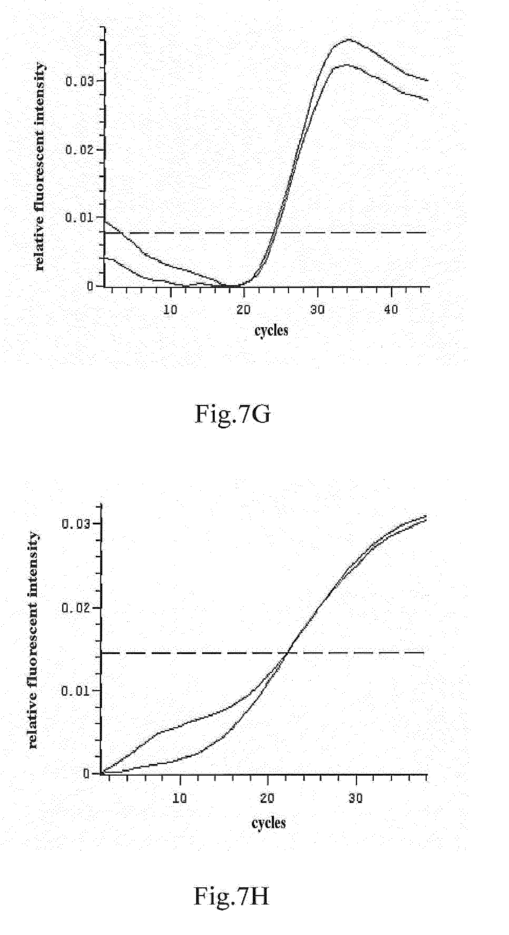

Figure 7 shows the amplification curve of fluorescent quantitative PCR of the wild-type (Fig.A) and T→G replacement (Fig.B) of EGFR Exon 21 position 2573 in a tissue sample; wild-type (Fig.C) and position 2235-2249 deletion (Fig.D) of EGFR Exon 19 in a tissue sample; EGFR Exon 19 position 2236-2250 deletion (Fig.E) in whole blood sample; EGFR position 2254-2277 deletion (Fig.F) in whole blood sample; and position 2155 wild-type (Fig.G) and G→A replacement (Fig.H) of EGFR Exon 18 in cell line sample.

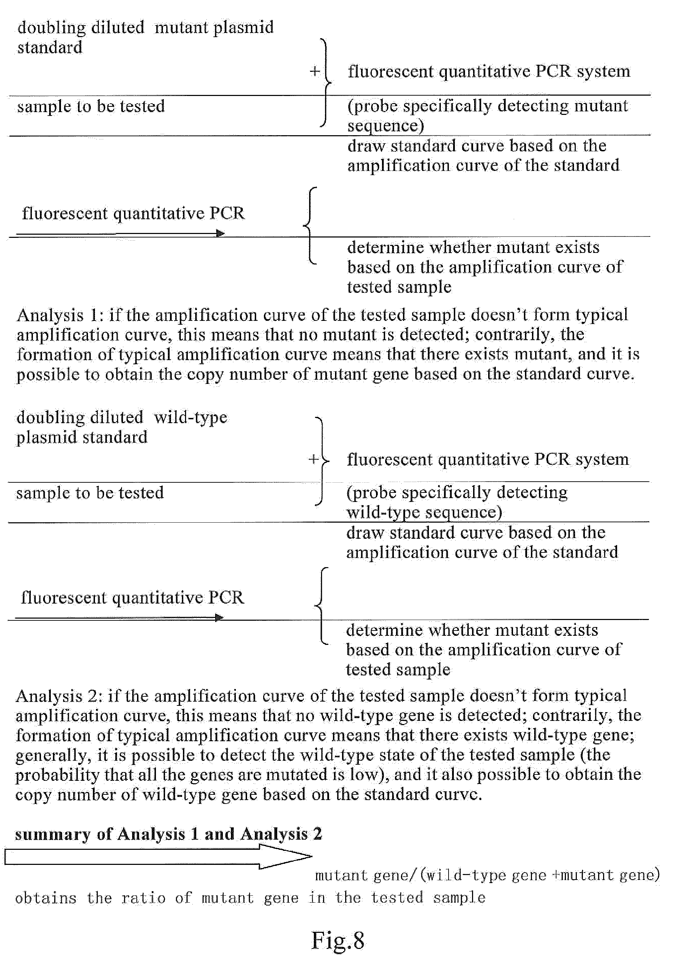

Figure 8 is a diagram of the quantitative method of the present invention.

### DETAILED DESCRIPTION OF PARTICULAR EMBODIMENTS

### Examples

The following examples are put forth so as to provide those of ordinary skill in the art with a complete disclosure and description of how to make and use the present invention, and are neither intended to limit the scope of what the inventors regard as their invention nor they intended to represent that the experiments below are all or the only experiments performed. The experiment conditions which are not indicated in the Examples, are generally conventional, such as those disclosed in "Molecular Cloning, A Laboratory Manual, 3^{rd} ed, (Sambrook J.)", or those suggested by the manufacture.

### Example 1: Extracting genome DNA from fresh human tumor tissues, paraffin embedded tissues, peripheral blood, pleural effusion, and human cell lines

The tumor cell lines we tested included cell lines of: non-small-cell carcinoma (NSCLC; A549, H460, H838 and H1703), breast cancer (MCF-7, BT474 and HuL100), malignant mesothelioma (H513, H2052, H290, MS-1 and H28), colon cancer (SW480), head and neck cancer (U87), cervical carcinoma (Hela), sarcoma (Mes-SA, Saos-2 and A204).

The fresh human tumor tissues, peripheral blood, paraffin embedded tissues we tested included: NSCLC, mesothelioma, colon cancer, malignant melanona, renal carcinoma, esophagus cancer, thyroid carcinoma, malignant cancer and ovarian cancer.

### Extraction of sample DNA

DNA extracting kit from Qiagen Inc., Promega Inc., or Roche Inc. can be used to extract genome DNA from the samples. Content and purity of the extracted DNA can be determined by using Nanodrop ND1000 (Gene Inc.) (OD260/OD280 is about 1.8, OD260/OD230 is more than 2.0). For example, the sample DNA may be extracted using the DNA Extracting Kit (Promega Inc.) as follows:
1. DNA extraction from fresh tissues
   (1) cut a bean-sized tissue using scissors, put it into a mortar, cut it into pieces, and ground it into powder by adding liquor nitrogen.
   (2) added 600µl pre-cooled lysate into the mortar, blow it 6 times using 1ml tip, sufficiently mixed the tissue powder and the lysate, transferred the mixture into a 1.5ml EP tube, then turned it over 6 times, water bath under 65°C for 20 minutes.
   (3) added 3µl RNase, turned over 6 times to mix homogenously, water bath under 37°C for 20 minutes.
   (4) cooled it to room temperature, added 200µl protein precipitation agent, turned over 6 time to mix homogenously, placed it on ice for 5 minutes, 13000 x g centrifuged 4 minute at room temperature.
   (5) transferred the supernatant into a new EP tube pre-added with 600µl isopropanol (room temperature), gently mixed 6 times, then 13,000 x g centrifugated at room temperate for 1 minutes.
   (6) discarded the supernatant, added 600µl 70% ethanol (room temperature) into precipitate, 13,000 x g centrifugated at room temperate for 1 minutes.
   (7) removed ethanol, air drying for 15 minutes.
   (8) added 40µl DNA solution into the precipitate, incubated at 65°C for 1 hour or 4°C overnight.
2. DNA extraction from paraffin embedded tissues
   (1) added 1mg or less tissues into 1.5ml centrifuge tube.
   (2) added freshly prepared 100µl incubation buffer/proteinase K solution, and incubated at 56°C overnight based on the type of the samples.
   (3) took out the incubated sample tube, added two times volume of lysate buffer.
   (4) vortex oscillated the resin for 10 seconds until the resin fully suspended, added 7µl fully suspended resin, vortex oscillated the resin for 3 seconds, then incubated at room temperature for 5 minutes.
   (5) vortex oscillated the resin for 2 seconds, put the tube on a magnetic separation rack (MagneSphere®), immediately conducted magnetic separation.
   (6) carefully removed all solution, without touching the resin on the tube wall.
   (7) added 100µl lysate buffer, got out the tube from the magnetic separation rack, vortex oscillate for 2 seconds.
   (8) put the tube back to the magnetic separation rack, removed all the lysate.
   (9) added 100µl 1x washing fluid, got out the tube from the magnetic separation rack, vortex oscillated 2 seconds.
   (10) put the tube back to the magnetic separation rack, removed all the lysate.
   (11) repeated step (9) and (10) twice, totally washed three times, and removed all the liquid after the last wash.
   (12) opened the lid, put the tube on the magnetic separation rack, air drying for 5 minutes.
   (13) added 25µl eluate.
   (14) closed the lid, vortex oscillate for 2 seconds, incubated at 65°C for 5 minutes.
   (15) took out the incubated tube, vortex oscillated for 2 seconds, immediately put it on the magnetic separation rack.
   (16) carefully transferred the DNA solution into a selected container.
3. DNA extraction of whole blood
   (1) obtained 300µl anticoagulant whole blood, added 900µl cell lysate, blow 6 times using 1ml tip, so that the whole blood and the cell lysate were sufficiently mixed, placed it under room temperature for 10 minutes, blew with the tip three times.
   (2) 13,000 x g centrifugated under room temperature for 20 seconds, discarded the supernatant, shook violently, added 300µl pre-cooling lysate, blew with 1ml tip until the precipitate were totally dissolved.
   (3) added 1.5µl RNase, turned over 6 times to mix homogenously, water bath under 37°C for 20 minutes.
   (4) cooled to room temperature, added 100µl protein precipitation agent, turned over 6 times to mix homogenously, placed it on ice for 5 minutes, 13,000 x g centrifugated under room temperature, for 4 minutes.
   (5) transferred the supernatant to a new EP tube previously added 300 µl isopropanol (room temperature), gently mixed 6 times, centrifugated under room temperature for 1 minutes.
   (6) discarded the supernatant, add 1 ml 70% ethanol (room temperature) into the precipitate, turned over 6 times to mix homogenously, 13,000 x g centrifugated under room temperature for 1 minutes.
   (7) removed ethanol, air drying for 15 minutes.
   (8) added 40µl DNA dissolving solution, stay at 65°C for 1 hour or 4°C overnight.
4. DNA extraction of pleural effusion,
   (1) obtained 5 ml pleural effusion, 2000 rpm centrifugated at room temperature for 10 minutes, removed the supernatant, added 1 ml cell lysate, turned over 6 times to mix homogenously, stayed under room temperature for 10 minutes.
   (2) 13,000 x g centrifugated under room temperature for 20 seconds, discarded the supernatant, shook violently, added 1 ml pre-cooling lysate, mixed until the precipitate totally dissolved.
   (3) added 3 µl RNase, turned over 6 times to mix homogenously, water bath under 37°C for 20 minutes.
   (4) cooled to room temperature, added 200µl protein precipitation agent, turned over 6 times to mix homogenously, placed it on ice for 5 minutes, 13,000 x g centrifugated under room temperature for 4 minutes.
   (5) transferred the supernatant to a new EP tube previously added 5 ml isopropanol (room temperature), gently mixed 6 times, 13,000 x g centrifugated under room temperature for 1 minutes.
   (6) discarded the supernatant, added 1 ml 70% ethanol (room temperature) into the precipitate, turned over 6 times to mix homogenously, 13,000 x g centrifugated under room temperature for 1 minutes.
   (7) sucked out ethanol, air drying for 15 minute.
   (8) added 40µl DNA dissolving solution, stay at 65°C for 1 hour or 4°C overnight.
5. DNA extraction from cell lines
   (1) obtained at least 1 x 10⁶ cells, transferred them into a 1.5 ml EP tube, 13,000 x g centrifugated at room temperature for 10 seconds. If the cells are adherent cells, they should be digested by trypsin before collecting them.
   (2) discarded the supernatant, added 200 µl PBS to wash the cells, 13,000 x g centrifugated under room temperature for 10 seconds, discarded the supernatant, shook violently until the precipitate was suspended.
   (3) added 600 µl pre-cooling lysis solution, blew to mix homogenously with 1 ml tip until no visual cell blocks.
   (4) added 3 µl RNase, turned over 6 times to mix homogenously, water bath under 37°C for 20 minutes.
   (5) cooled to room temperature, added 200µl protein precipitation agent, turned over 6 times to mix homogenously, placed it on ice for 5 minutes, 13,000 x g centrifugated under room temperature for 4 minutes.
   (6) transferred the supernatant to a new EP tube previously added 600µl isopropanol (room temperature), gently mixed 6 times, 13,000 x g centrifugated under room temperature for 1 minutes.
   (7) discarded the supernatant, added 600 µl 70% ethanol (room temperature) into the precipitate, turned over 6 times to mix homogenously, 13,000 x g centrifugated under room temperature for 1 minutes.
   (8) sucked out ethanol, air drying for 15 minutes.
   (9) added 40µl DNA dissolving solution, stayed at 65°C for 1 hour or 4°C overnight.

### Example 2: Preparation of the plasmid standards containing mutant and wild-type sequences

### 1. Construction of wild-type plasmids (Figure 1, Figure 2)

### 1.1 Preparation of the carrier

TA cloning carrier pMD18-T was purchased from TAKARA Inc.

### 1.2 Preparation of the insert

The insert is prepared using PCR. The template of PCR is the sample genome DNA extracted in Step 1. The reaction system and amplification condition are shown in the following tables (Table 1, Table 2 and Table 3):

**Table 1: PCR reaction system (50µl)**

| reagents | amount(µl/tube) |
|---|---|
| double-distilled water | 29.75 |
| 10x buffer (free of Mg²⁺) | 5 |
| MgCl₂ (25mM) | 7.5 |
| dNTP (10mM) | 1.25 |
| upstream primer (25µM) | 1.25 |
| downstream primer (25µM) | 1.25 |
| Taq enzyme | 1 |
| DNA template | 3 |
| total volume | 50 |

For preparing the plasmid containing wild-type sequence at EGFR gene Exon 18 position 2155, it needs to add the primer sequences of E18-F-1 (SEQ ID NO:5) or E18-F-2 (SEQ ID NO:6) and E18-R-1 (SEQ ID NO:7) or E18-R-2 (SEQ ID NO:8) into the amplification system. For preparing the plasmid containing wild-type sequence of EGFR gene Exon 19 positions 2235-2249, 2236-2250 and 2254-2277, it needs to add the primer sequences of E19-F-1 (SEQ ID NO:9) or E19-F-2 (SEQ ID NO:10) and E19-R-1 (SEQ ID NO:11) or E19-R-2 (SEQ ID NO:12). For preparing the plasmid containing wild-type sequence at EGFR gene Exon 21 positions 2573, it needs to add the primer sequences of E21-F-1 (SEQ ID NO: 13) or E21-F-2 (SEQ ID NO:14) and E21-R-1 (SEQ ID NO:15) or E21-R-2 (SEQ ID NO: 16).

**Table 2: PCR primers**

| name | sequence | |
|---|---|---|
| E18-F-1 | GAGGATCTTGAAGGAAACTG | ( SEQ ID NO:5 ) |
| E18-F-2 | CCAGCTTGTGGAGCCTCTT | ( SEQ ID NO:6 ) |
| E18-R-1 | GCCAGGGACCTTACCTTAT | ( SEQ ID NO:7 ) |
| E18-R-2 | CTGTGCCAGGGACCTTACCTT | ( SEQ ID NO:8 ) |
| E19-F-1 | CCCAGAAGGTGAGAAAGTT | ( SEQ ID NO:9 ) |
| E19-F-2 | GGGACTCTGGATCCCAGAAG | (SEQ ID NO:10) |
| E19-R-1 | CCTGAGGTTCAGAGCCAT | (SEQ ID NO:11) |
| E19-R-2 | CCCACACAGCAAAGCAGAA | (SEQ ID NO:12) |
| E21-F-1 | GCAGCCAGGAACGTACTGGT | (SEQ ID NO:13) |
| E21-F-2 | CCCTCACAGCAGGGTCTTCT | (SEQ ID NO:14) |
| E21-R-1 | GTGGGAAGGCAGCCTGGT | (SEQ ID NO:15) |
| E21-R-2 | GTGGGAAGGCAGCCTGGT | (SEQ ID NO:16) |

**Table 3 : PCR amplification condition**

| steps | cycles | |
|---|---|---|
| Step 1 | 1 | 95°C , 1-5 minutes |
| Step 2 | 20-30 | 95°C, 10-15 seconds ; 55-65°C, 30-60 seconds |

1.3 After recovering the target fragment using QIAgen Gel Recover Kit, inserted said fragment into pMD18-T (purchased from TAKARA Inc.) by TA colonizing.

1.4 Amplified the constructed plasmid in E. coli DH5α strain, and harvested by extraction and purification (the methods are showed in Molecular Cloning, A Laboratory Manual, 3rd ed. pages 96-99 and 103.

1.5 Identified the plasmid by double enzyme digestion of BamHI and HindIII.

1.6 Sequenced the strains having positive result, and used the strains with correct sequence as the standard containing wild-type sequence (Figure 3).

2. Construction of mutant plasmids: designed mutant primers of mutant sites, obtained the standards containing mutant sequences by DPN method.

2.1 Designed the mutant primers (Figure 4) of mutant sites based on the desired mutant sequences.

**Table 4 : mutant primers**

| primers name | sequences | |
|---|---|---|
| E18-M-F: | TGCTGAGCTCCGGTGCGTTCG | (SEQ ID NO:17) |
| E18-M-R: | GGAGCTCAGCACTTTGATCTT | (SEQ ID NO:18) |
| E19-1-F: | ATCAAAACATCTCCGAAAGCC | (SEQ ID NO:19) |
| E19-1-R: | ATGTTTTGATAGCGACGGGAA | (SEQ ID NO:20) |
| E19-2-F: | TCAAGACATCTCCGAAAGCCA | (SEQ ID NO:21) |
| E19-2-R: | GATGTCTTGATAGCGACGGGA | (SEQ ID NO:22) |
| E19-3-F: | CAACACTCGATGTGAGTTTCT | (SEQ ID NO:23) |
| E19-3-R: | TCGAGTGTTGCTTCTCTTAAT | (SEQ ID NO:24) |
| E21-M-F: | TGGGCGGGCCAAACTGCTGGG | (SEQ ID NO:25) |
| E21-M-R: | TGGCCCGCCCAAAATCTGTGA | (SEQ ID NO:26) |

2.2 Used 5ng wild-type plasmid as template, and used mutant primers and Pfu enzyme to mutate the target sites. The amplification system and condition are shown in Table 1, Table 4 and Table 3.

During the preparation of the plasmid containing the mutant sequence with EGFR gene Exon 18 position 2155 G→A, it needs to add E18-M-F (SEX ID NO:17) and E18-M-R (SEQ ID NO:18) primers into the amplification system. During the preparation of the plasmid containing the mutant sequence with EGFR gene Exon 19 position 2235-2249 deletion, it needs to add E19-1-F (SEQ ID NO:19) and E19-1-R (SEQ ID NO:20) primers into the amplification system. During the preparation of the plasmid containing the mutant sequence with EGFR gene Exon 19 position 2236-2250 deletion, it needs to add E19-2-F (SEQ ID NO:21) and E19-2-R (SEQ ID NO:22) primers into the amplification system. During the preparation of the plasmid containing the mutant sequence with EGFR gene Exon 19 position 2254-2277 deletion, it needs to add E19-3-F (SEQ ID NO:23) and E19-3-R (SEQ ID NO:24) primers into the amplification system. During the preparation of the plasmid containing the mutant sequence with EGFR gene Exon 21 position 2573 T→G, it needs to add E21-M-F (SEQ ID NO:25) and E21-M-R primer (SEQ ID NO:26) primers into the amplification system.

2.3 treated the product obtained in step 2.2 with DPN1 enzyme, recovered the product after incubating at 37°C for 1 hour, amplified in E. coli DH5α strain, and harvested by extraction and purification.

2.4 Identified the plasmid by double enzyme digestion of BamHI and HindIII.

2.5 Sequenced the strains having positive result, and used the strains with correct sequence as the standard containing mutant sequence (Figure 4).

### Example 3: Detection of EGFR mutations from genome DNA of human cell lines, human fresh tumor tissues, peripheral blood, and paraffin embedded tissues, using lung cancer and cervical carcinoma as examples.

1. The templates for fluorescent quantitative PCR were the genome DNA of lung cancer and cervical carcinoma samples extracted in Example 1, and the standards prepared in Example 2. Double-distilled water was served as negative control. For drawing the standard curves, the standards were diluted as 1ng/µl , 0.5ng/µl. 0.25ng/µl, 0.125ng/µl, 0.0625ng/µl, 0.03125ng/µl.

2. The reaction system and condition are shown in Table 2, Table 5, Table 6 and Table 7, wherein the fluorescent emission group bound to the probe is selected from FAM, TET, HEX or ROX, the quench group is selected from BHQ or TAMARA.

**Table 5 : Reaction system for fluorescent quantitative PCR (20µl/tube)**

| reagent | amount (µl/tube) |
|---|---|
| double-distilled water | 9.9 |
| 10×buffer (free of Mg²⁺) | 2 |
| MgCl₂ (25mM) | 3 |
| dNTP (10 mM) | 0.5 |
| upstream primer (25_{µ}M) | 0.5 |
| downstream primer ( 25µM ) | 0.5 |
| fluorescent probe ( 25µM ) | 0.2 |
| Taq enzyme | 0.4 |
| DNA template | 3 |
| total volume | 20 |

For detecting the G→A mutation at EGFR gene Exon 18 position 2155, it needs to prepare two systems, i.e. separately add E18-F-1 (SEQ ID NO:5) and E18-F-2 (SEQ ID NO:6) and E18-R-1 (SEQ ID NO:7) or E18-R-2 (SEQ ID NO:8) primers, E18W-1 (SEQ ID NO:27) or E18W-2 (SEQ ID NO:28) probe, E18-F-1 (SEQ ID NO:5) or E18-F-2 (SEQ ID NO:6) and E18-R-1 (SEQ ID NO:7) or E18-R-2 (SEQ ID NO:8) primers, E18M-1 (SEQ ID NO:29) or E18W-2 (SEQ ID NO:30) probe.

For detecting the Exon 19 deletions (positions 2235-2249, 2236-2250 and 2254-2277), it needs to prepare 4 systems, i.e. separately add E19-F-1 (SEQ ID NO:9) or E19-F-2 (SEQ ID NO:10) and E19-R-1 (SEQ ID NO:11) or E19-R-2 (SEQ ID NO:12) primers, E19W-1 (SEQ ID NO:31) or E19W-2 (SEQ ID NO:32) probe ; E19-F-1 (SEQ ID NO:9) or E19-F-2 (SEQ ID NO:10) and E19-R-1 (SEQ ID NO:11) or E19-R-2 (SEQ ID NO:12) primers, E19M1-1 (SEQ ID NO:33) or E19M1-2 (SEQ ID NO:34) probe, E19-F-1 (SEQ ID NO:9) or E19-F-2 (SEQ ID NO:10) and E19-R-1 (SEQ ID NO:11) or E19-R-2 (SEQ ID NO:12) primers, E19M2-1 probe (SEQ ID NO:35) or E19M2-2 (SEQ ID NO:36); E19-F-1 (SEQ ID NO:9) or E19-F-2 (SEQ ID NO:10) or E19-R-1 (SEQ ID NO:11) or E19-R-2 (SEQ ID NO:12) primers, E19M3-1 (SEQ ID NO:37) or E19M3-2 (SEQ ID NO:38) probe. For detecting the T→G mutation at Exon 21 position 2573, it needs to prepare two systems, i.e., separately add E21-F-1 (SEQ ID NO:13) or E21-F-2 (SEQ ID NO:14) and E21-R-1 (SEQ ID NO:15) or E21-R-2 (SEQ ID NO:16) primers, E21W-1 (SEQ ID NO:39) or E21W-2 (SEQ ID NO:40) probe, 21-F-1 (SEQ ID NO:13) or E21-F-2 (SEQ ID NO:14) and E21-R-1 (SEQ ID NO:15) or E21-R-2 (SEQ ID NO:16) primers, E21M-1 (SEQ ID NO:41) or E21M-2 (SEQ ID NO:42) probe.

**Table 6 : Probes**

| names | sequences | |
|---|---|---|
| E18W-1 | GGCTCCGGTGCGTTCGGC | (SEQ ID NO:27) |
| E18W-2 | CGGAGCCCAGCACTTTGATCT | ( SEQ ID NO:28 ) |
| E18M-1 | TGCTGAGCTCCGGTGCGTT | ( (SEQ ID Neo:29 |
| E18M-2 | CGGAGCTCAGCACTTTGATCTT | ( SEQ ID NO:30 ) |
| E19W-1 | TCAAGGAATTAAGAGAAGCAACATC | ( SEQ ID NO:31 ) |
| E19W-2 | CGGAGATGTTGCTTCTCTTAATTCCT | ( SEQ ID NO:32 ) |
| E19M1-1 | CCCGTCGCTATCAAAACATCT | ( SEQ ID NO:33 ) |
| E19M1-2 | AGATGTTTTGATAGCGACGGG | ( SEQ ID NO:34 ) |
| E19M2-1 | CCCGTCGCTATCAAGACATCTC | ( SEQ ID NO:35 ) |
| E19M2-2 | GAGATGTCTTGATAGCGACGGG | ( SEQ ID NO:36 ) |
| E19M3-1 | AATTAAGAGAAGCAACACTCGAT | ( SEQ ID NO:37 ) |
| E19M3-1 | ATCGAGTGTTGCTTCTCTTAATT | ( SEQ ID NO:38 ) |
| E21W-1 | TGGCCAGCCCAAAATCTGTG | ( SEQ ID NO:39 ) |
| E21W-1 | AAGATCACAGATTTTGGGCTGGC | ( SEQ ID NO:40 ) |
| E21M-1 | TGGCCCGCCCAAAATCTGT | ( SEQ ID NO:41 ) |
| E21M-1 | GATCACAGATTTTGGGCGGGC | ( SEQ ID NO:42 ) |

**Table 7 : Amplification condition**

| steps | cycles | |
|---|---|---|
| step | 1 | 95°C, 1-5minutes |
| step 2 | 30-45 95°C, | 10-15 seconds ; 55-65°C (collect fluorescent), 30-60 seconds |

### 3. Drawing the standard curve

The standard curve was drawn based on the CT values obtained from the standard in Step 3. Figure 5 shows the amplification curve of plasmid standard, in which the five rising curves represent, from left to right, the amplification curve of the plasmid standard with the dilute ratio of 0.5ng/µl, 0.25ng/µl, 0.125ng/µl, 0.0625ng/µl and 0.03125ng/µl respectively. The horizontal axis represents cycle number, and the vertical axis represents fluorescent detection value. Accordingly, it is possible to draw the standard curve for calculation (Figure 6). In Figure 6, the horizontal axis represents the logarithm of copy number of the template, the vertical axis represents CT value, wherein the copy number of template=mass/(molecular weight)×6.02×10²³, the molecular weight of plasmid≈the number of bases ×324.5, or is calculated using the software DNAMAN. In the present experiment, the plasmid is consisted of pMD18-T carrier and an insert. Because the lengths of the insert are almost identical, the biggest difference only lies in twenties bases, which can be ignored with respect to the length of 2692bp for PMD18-T carrier. Therefore, the ratio of copies of wild-type to mutant plasmid standard ≈the ratio of weight. The molecular weight of the plasmid of the present experiment≈890 ∼ 900Kda, so the copy number of 1ng plasmid is about 6.69×10⁸∼ 6.76×10⁸.

### 4. Calculation of the ratio of specific EGFR mutation in a sample

According to the standard curve, the copy numbers of wild-type and mutant genome DNA were calculated from the CT values of the sample. Then we obtained the ratio of mutant EGFR DNA to total EGFR DNA (wild-type plus all mutants at said site). As shown in Figure 7, the wild-type CT value of EGFR Exon 21 of a tissue sample was 19.15, whereas the value of T→G mutation at position 2573 was 20.74. According to each standard curve formula (Figure 6), we could calculate the copy numbers for them. We then obtained the ratio of the content of mutant to wild-type which was 89:100, and we estimated that about 47% EGFR gene in the tissue sample had T→G mutation at position 2573.

### 5. Result of detection

In this Example, we detected the EGFR gene mutation in 48 cases of tissues, whole blood and cell line samples of lung cancer and cervical carcinoma, and found that 13 cases had mutations, and the concrete number can be seen in Table 8. The mutation ratios, i.e. the ratio of mutant gene to non-mutant gene in those samples, can be seen in Table 9.

**Table 8 : EGFR mutant cases**

| mutation type | case number |
|---|---|
| Exon 18 2155 G→A | 1 |
| Exon 19 2235→2249Del | 3 |
| Exon 19 2236→2250Del | 1 |
| Exon 19 2254→2277Del | 2 |
| Exon 21 L858R | 6 |
| total | 13 |

**Table 9 : EGFR mutation ratio**

| type of mutant samples | mutation type | mutation ratio |
|---|---|---|
| H1975 cell line | Exon 21 L858R | 25% |
| cervical carcinoma tissue | Exon 19 2235→2249Del | 30% |
| | Exon 19 2254→2277Del | 15% |
| lung cancer tissue | Exon 18 2155 G→A | 32% |
| | Exon 19 2235→2249Del | 18% |
| | Exon 19 2236→2250Del | 28% |
| | Exon 19 2254→2277Del | 15% |
| | Exon 21 L858R | 10%, 12%, 15%, 15%, 47% |
| cervical carcinoma whole blood | Exon 19 2235→2249Del | 25% |

### Example 4: Detection ofEGFR mutations from mRNA of human cell lines, human fresh tumor tissues, peripheral blood, and paraffin embedded tissues

### 1. Extracted total RNA from samples

Use Total RNA Extraction Kit (Invotrogen Inc., OMEGA Inc.) to extract RNA from the samples, and use Nanodrop ND1000 spectrophotometer (Gene Inc.) to detect the concentration and purification of the RNA (OD260/280 is between 1.8-2.0, OD260/230>2.0).

### 2. Use M-MLV reverse-transcripatase to conduct reverse transcription. The steps and reaction system are shown in Table 10:

**Table 10 : reverse transcription system (10µl) and reaction steps**

| reagent | amount ( µl/tube ) |
|---|---|
| RNA template | 5.5 |
| OligodT | 0.4 |
| 70°C denatured 5min, ice incubation 2-5min, add the following agent | |
| 5×buffer | 2 |
| dNTP(5mM) | 1 |
| DEPC water | 0.35 |
| RNasin(40U) | 0.25 |
| MLV RT-enzyme | 0.5 |
| Total | 10 |

| | |
|---|---|
| 37-42°C 60-90min, 70°C 5min. | |

3. The preparation method of the standards and the steps of real-time fluorescent PCR detection were similar to those in Example 1. The primers for preparing wild-type plasmid standards and the probes for fluorescent quantitative PCR detection were the same with those in Example 1, except that the primers for preparing wild-type plasmid standards and fluorescent quantitative PCR detection were different (Table 11). The method for drawing standard curve and calculating the content of specific mutations of EGFR gene were the same with those in Example 1.

**Table 11 : The primers for preparing wild-type plasmid standards and for fluorescent quantitative PCR detection of the samples when using cDNA as template**

| name | sequence | |
|---|---|---|
| E18-F-1 | GAGGATCTTGAAGGAAACTG | ( SEQ ID NO:5 ) |
| E18-R-3 | TGGGATCCAGAGTCCCTT | ( SEQ ID NO:43 ) |
| E19-F-1 | CCCAGAAGGTGAGAAAGTT | ( SEQ ID NO:9 ) |
| E19-R-3 | GTCTTTGTGTTCCCGGACAT | ( SEQ ID NO:44 ) |
| E21-F-1 | GCAGCCAGGAACGTACTGGT | ( SEQ ID NO: 13 ) |
| E21-R-3 | GCCTCCTTCTGCATGGTATT | ( SEQ ID NO:45 ) |

### Choose the primers for preparing wild-type plasmid standards:

For preparing the plasmid containing EGFR gene Exon 18 wild-type sequence at position 2155, it needs to add E18-F-1 (SEQ ID NO:5) and E18-R-3 (SEQ ID NO:43) primers. For preparing the plasmid containing EGFR gene Exon 19 wild-type sequences at positions 2235-2249, 2236-2250 and 2254-2277, it needs to add E19-F-1 (SEQ ID NO:9) and E19-R-3 (SEQ ID NO:44). For preparing the plasmid containing EGFR gene Exon 21 wild-type sequences at position 2573, it needs to add E21-F-1 (SEQ ID NO: 13) and E21-R-2 (SEQ ID NO:45) primers.

### Choose the primers and probes for fluorescent quantitative PCR detection:

For detecting the G→A mutation at EGFR gene Exon 18 position 2155, it needs to prepare two systems, i.e. separately add E18-F-1 (SEQ ID NO:5) and E18-R-3 (SEQ ID NO:43) primers, E18W-1 (SEQ ID NO:27) or E18W-2 (SEQ ID NO:28) probe, E18-F-1 (SEQ ID NO:5) and E18-R-3 (SEQ ID NO:43) primers, E18M-1 (SEQ ID NO:29) or E18W-2 (SEQ ID NO:30) probe.

For detecting the deletions in Exon 19 at positions of 2235-2249, 2236-2250, and 2254-2277, it needs to prepare 4 systems, i.e., separately add E19-F-1 (SEQ ID NO:9) and E19-R-3 (SEQ ID NO:44) primers, E19W-1 (SEQ ID NO:31) or E19W-2 (SEQ ID NO:32) probe, E19-F-1 (SEQ ID NO:9) and E19-R-3 (SEQ ID NO:44) primers, E19M1-1 (SEQ ID NO:33) or E19M1-2 (SEQ ID NO:34) probe, E19-F-1 (SEQ ID NO:9) and E19-R-3 (SEQ ID NO:44) primers, E19M2-1 probe (SEQ ID NO:35) or E19M2-2 (SEQ ID NO:36), E19-F-1 (SEQ ID NO:9) and E19-R-3 (SEQ ID NO:44) primers, E19M3-1 (SEQ ID NO:37) or E19M3-2 (SEQ ID NO:38) probe.

For detecting the T→G mutation at Exon 21 position 2573, it needs to prepare two systems, i.e. separately add E21-F-1 (SEQ ID NO:13) and E21-R-3 (SEQ ID NO:45) primers, E21W-1 (SEQ ID NO:39) or E21W-2 (SEQ ID NO:40) probe ; E21-F-1 (SEQ ID NO:13) and E21-R-3 (SEQ ID NO:45) primers, E21M-1 (SEQ ID NO:41) or E21M-2 (SEQ ID NO:42) probe.

## Claims

1. A PCR primer, **characterized in that** it binds with nucleotides within a sequence under a suitable PCR condition, said sequence having 200 bases and comprising a mutation site of the EGFR gene.

2. The primer according to claim 1, **characterized in that** said primer is consisted of upstream primer and downstream primer.

3. A probe for fluorescent quantitative PCR, **characterized in that** it specifically bind to the base sequence at EGFR gene mutant site under suitable PCR condition.

4. A probe according to claim 3, **characterized in that** said probe is linked to a fluorescent emitting group at its 5' end, and is linked to a fluorescent quench group at its 3' end.

5. A plasmid containing EGFR gene wild-type sequence to be tested.

6. A plasmid according to claim 5, **characterized in that** the wild-type sequence of EGFR gene is SEQ ID NO:46, SEQ ID NO:48 or SEQ ID NO:52.

7. A plasmid containing mutant sequence of EGFR gene to be tested.

8. The plasmid according to claim 7 **characterized in that** the EGFR gene mutant sequence contained in the plasmid is SEQ ID NO:47, SEQ ID NO:49, SEQ ID NO:50, SEQ ID NO:51 or SEQ ID NO:53,

9. A quantitative detection kit for quantitatively detecting EGFR gene mutations, **characterized in that** it includes: the primers according to claim 1 or 2, the probes according to claim 3 or 4, and the plasmids according to claim 5 and/or 7.

10. A kit according to claim 9 **characterized in that** the ratio of said primer to probe is 2:1-10:1, and said primer comprises a forward primer and reverse primer in a ratio of 1:3-3:1.
